(19) Europäisches Patentamt
European Patent Office
Office européen des brevets

(11) Publication number: 0 473 108 A2

# (12) EUROPEAN PATENT APPLICATION

(21) Application number: 91114345.1

(51) Int. Cl.$^5$: A61K 31/70

(22) Date of filing: 27.08.91

(30) Priority: 30.08.90 JP 229315/90

(43) Date of publication of application: 04.03.92 Bulletin 92/10

(84) Designated Contracting States: AT BE CH DE DK ES FR GB GR IT LI LU NL SE

(71) Applicant: MECT CORPORATION
2-1-1, Nishi-Shinjyuku Shinjyuku-ku
Tokyo(JP)

(72) Inventor: Ishii, Takayuki
c/o MECT Corporation, 1-1, Nishi-Shinjuku 2-chome
Shinjuku-ku, Tokyo(JP)
Inventor: Kasano, Makiko
c/o MECT Corporation, 1-1, Nishi-Shinjuku 2-chome
Shinjuku-ku, Tokyo(JP)
Inventor: Yasunaga, Tae
c/o MECT Corporation, 1-1, Nishi-Shinjuku 2-chome
Shinjuku-ku, Tokyo(JP)
Inventor: Ishii, Mariko
c/o MECT Corporation, 1-1, Nishi-Shinjuku 2-chome
Shinjuku-ku, Tokyo(JP)
Inventor: Sugimoto, Mamoru
c/o MECT Corporation, 1-1, Nishi-Shinjuku 2-chome
Shinjuku-ku, Tokyo(JP)
Inventor: Tomita, Kenkichi
c/o MECT Corporation, 1-1, Nishi-Shinjuku 2-chome
Shinjuku-ku, Tokyo(JP)
Inventor: Miyata, Takeshi
2178-64, Nagamine-machi
Kumamoto-shi, Kumamoto(JP)
Inventor: Tanaka, Makoto
c/o MECT Corporation, 1-1, Nishi-Shinjuku 2-chome
Shinjuku-ku, Tokyo(JP)

(74) Representative: Leyh, Hans, Dr.-Ing. et al
Patentanwälte Berendt, Leyh & Hering Innere Wiener Strasse 20
W-8000 München 80(DE)

## (54) Preparation for treating renal disease.

(57) A preparation for treating renal disease, such as nephiritis, which comprises, an effective amount of N-acetylneuraminic acid shown by the formula:

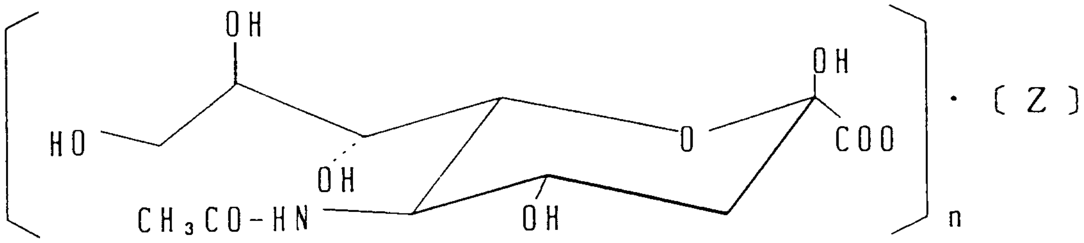

wherein, when n is 1, Z represents hydrogen, lithium, potassium, sodium, ammonium or oraganic ammonium, and, when n is 2, Z represents calcium, barium or magnesium; and, a pharmacologically acceptable carrier.

EP 0 473 108 A2

FIG. 1

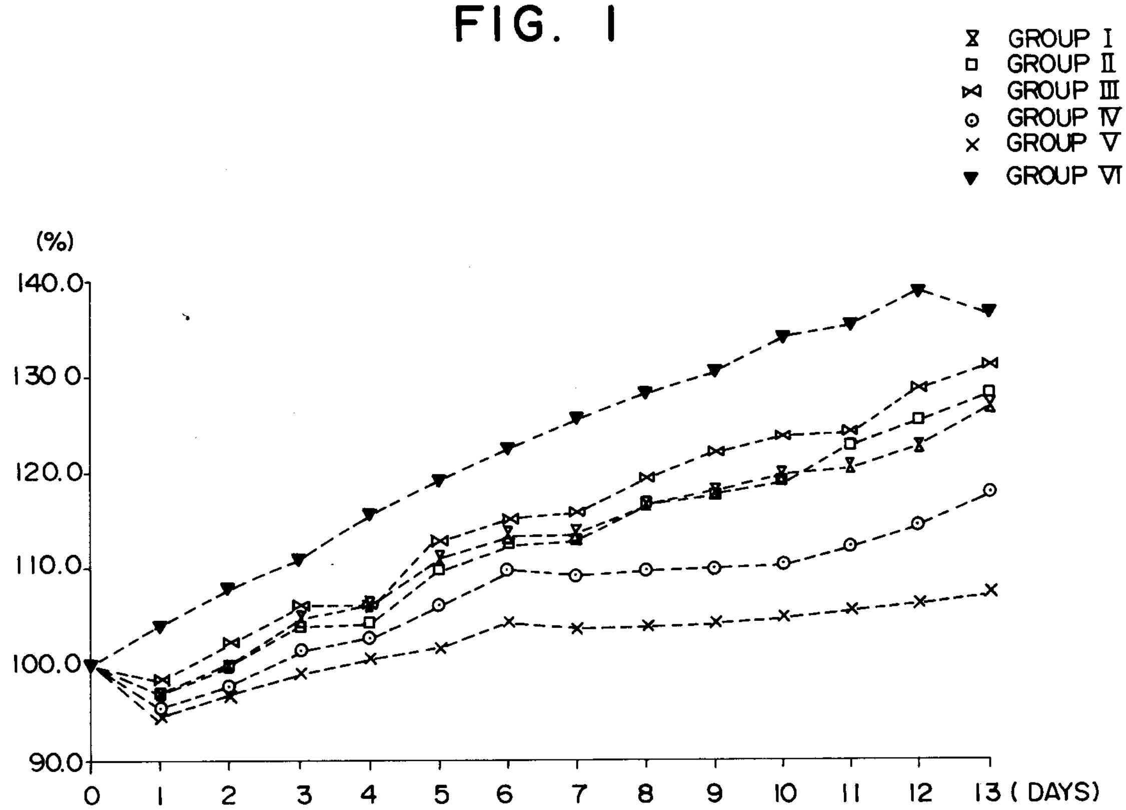


WEIGHT GAIN RATE

## BACKGROUND OF THE INVENTION

### 1. Field of the Invention

This invention relates to a preparation for treating renal disease, containing N-acetylneuraminic acid or its salt as an effective ingredient.

### 2. Related Art Statement

N-acetylneuraminic acid has been reported to exhibit antiviral action, antiinflammatory action and antiallergic action ((1) Hess, E.L. et al, The Journal of Immunology, Vol.127, No.5 (1981): 1740-1743, (2) Görög, P. et al, Agents and Actions, Vol.8, No.5 (1978): 543-545, (3) Ito, H., Yakuri to Chiryo (Japanese), Vol.13, No.7 (1985): 479-494, (4) The 61st General Conference of Japan Pharmacology Society (March 23-26, 1988, (Antiinflammatory action of N-acetylneuraminic acid (NANA) in guinea pigs(Japanese)).

The inventors have found inhibitory effect of N-acetylneuraminic acid on renal disease, which action had not been known, leading to the present invention.

## SUMMARY OF THE INVENTION

An object of the invention is to provide a preparation for treating renal disease, containing N-acetylneuraminic acid as an effective ingredient.

A preparation for treating renal disease according to the invention contains, as an effective ingredient, N-acetylneuraminic acid shown by the following formula:

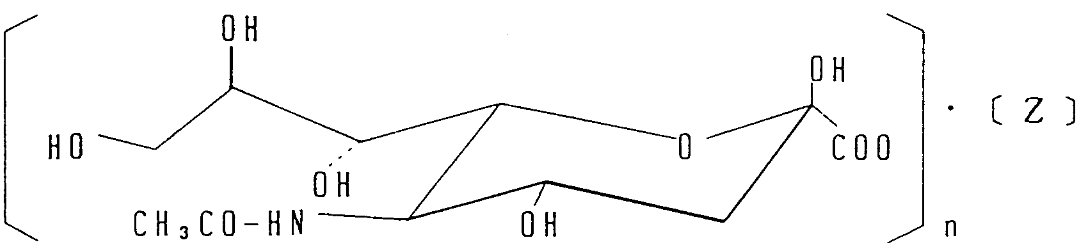

where, at n = 1, Z represents hydrogen, lithium, potassium, sodium, ammonium or organic ammonium, and, at n = 2, z represents calcium, barium or magnesium,
or salt of N-acetylneuraminic acid.

The preparation according to the invention may contain pharmacologically acceptable carrier such as physiological saline solution, stabilizers or preservatives.

The preparation may be administered intraperitoneally, subcutaneously or intravenously by injection.

The inhibitory effect of the compound on nephritis is explained by following examples, referring to the attached drawings.

## BRIEF DESCRIPTION OF THE DRAWINGS

Fig. 1 is a graph showing weight gain rates of experimental animals,
Fig. 2 is a graph showing kidney moist weights of experimental animals, and
Fig. 3 is a graph showing changes in urinary protein contents of experimental animals.

## EXAMPLES

[Example 1]

Effect on Puromycin aminonucleoside(PAN)-induced nephritis:

(1) Experimental animals

Male Sprague-Dawley(SD) rats weighing 160-180 g were used.

(2) Materials

As the nephritis causing substance, PAN (made by Sigma Co., Ltd.) was used. As the test compound, sodium N-acetylneuraminate (made by MECT Corporation) was used.

(3) Methods

PAN was intraperitoneally administered in amount of 80 mg per 1 kg of body weight to rats to cause nephritis. Then, the test compound (Groups I, II or III) or physiological saline solution (Group IV) was intraperitoneally administered 3 times a day (morning, noon and evening) for consecutive 14 days immediately after treatment with PAN as follows. As a control, methyiprednisolone was subcutaneously administered to rats of Group V once a day (noon) for consecutive 14 days immediately after treatment with PAN. Moreover, 3, 6, 9 and 13 days after treatment with PAN, 5 ml water was loaded, and the urine was collected for 6 hours. Fourteen days after treatment with PAN, autopsies were made, the kidneys were extirpated and their moist weights were measured.

Group I: 0.1 mg Sodium N-acetylneuraminate per 1 kg of body weight was administered.
Group II: 0.5 mg Sodium N-acetylneuraminate per 1 kg of body weight was administered.
Group III: 1.0 mg Sodium N-acetylneuraminate per 1 kg of body weight was administered.
Group IV: 2.5 ml Physiological saline solution per 1 kg of body weight was administered.
Group V: 2 mg Methylprednisolone per 1 kg of body weight was administered.
Group VI: Normal rats (no drug was given).

(4) Results

[1] The weight gain rates in Group I-VI are shown in Table 1 and Fig. 1 and the moist kidney weights in Table 1 and Fig. 2.

Table 1

| Experimental group | Number of rats | Weight gain rate (%) | Kidney moist Weight (g/100g) |
|---|---|---|---|
| I (Sodium N-acetylneuraminate 0.1 mg/kg) | 10 | 127.1±2.0 | 0.47±0.02 |
| II (Sodium N-acetylneuraminate 0.5 mg/kg) | 10 | 128.4±1.8 | 0.44±0.02 |
| III(Sodium N-acetylneuraminate 1.0 mg/kg) | 10 | 131.3±1.9 | 0.42±0.02 |
| IV (Physiological saline solution) | 10 | 118.1±1.6 | 0.48±0.01 |
| V (Methylprednisolone 2.0 mg/kg) | 10 | 107.7±1.6 | 0.43±0.01 |
| VI (Normal animals) | 10 | 134.7±2.8 | 0.37±0.01 |

Although weight gain was inhibited by administration of PAN (Group IV), the groups treated with sodium N-acetylneuraminate showed dose-dependent prevention against inhibition of weight gain and an improvement in systemic symptoms (Groups I-III). In addition, kidney moist weight was increased after administration of PAN (Group IV), while the increase was dose-dependently inhibited in the groups treated with sodium N-acetylneuraminate (Groups I-III). Also in the group given methylprednisolone as a positive control (Group V), similar inhibitory effect was observed.

[2] The urinary portein contents on the 3rd, 6th, 9th and 13th day in Groups I-VI are shown in Table 2 and Fig. 3.

Table 2

| Experimental group | Number of rats | Urinary protein content (mg/24 hours) | | | |
|---|---|---|---|---|---|
| | | 3rd day | 6th day | 9th day | 13th day |
| I (Sodium N-acetylneuraminate 0.1 mg/kg) | 10 | 14.1±1.1 | 83.4±8.6 | 99.6±14.2 | 109.9±12.9 |
| II (Sodium N-acetylneuraminate 0.5 mg/kg) | 10 | 11.3±0.8 | 49.1±13.6 | 59.9±14.7 | 94.5±30.4 |
| III(Sodium N-acetylneuraminate 1.0 mg/kg) | 10 | 5.1±0.4 | 50.5±10.7 | 51.2±15.6 | 77.9±17.9 |
| IV (Physiological saline solution) | 10 | 9.5±2.1 | 109.5±17.7 | 145.9±29.1 | 236.4±43.6 |
| V (Methylprednisolone 2.0 mg/kg) | 10 | 22.3±2.1 | 55.8±11.4 | 68.7±13.1 | 56.1±12.5 |
| VI (Normal animals) | 10 | 6.5±1.7 | 11.3±0.3 | 15.7±2.5 | 16.3±1.7 |

Administration of PAN increased the urinary protein content from the 6th day (Group IV), while, in the groups given sodium N-acetylneuraminate (Groups I-III), increases in the urinary protein contents were dose-dependently inhibited on the 6th, 9th and 13th day. The group given methylprednisolone as a positive control (Group V) also showed similar inhibitory effect.

(5) Judgement

The above-mentioned results on PAN-induced nephritis indicate that sodium N-acetylneuraminate has a therapeutic effect on renal disease.

[Example 2]

Acute toxicity test:

Acute toxicity tests of sodium N-acetylneuraminate in mice, rats and guinea pigs by oral adimnistration, subcutaneous injection, intraperitoneal injection, intravenous injection and inhalation were performed as follows.

(1) Experimental animals

| | |
|---|---|
| ICR mice<br>SD rats<br>Hartley guinea pigs | 6 weeks of age<br>6 weeks of age<br>6 weeks of age |

(2) Drug concentration

20% (w/v) dissolved in distilled water

(3) The number of animals at each level

10 mice, rats or guinea pigs

(4) Period of observation

14 days

(5) Calculation of $LD_{50}$

Probit method
The results are shown in Table 3.

Table 3

Acute toxicity test of sodium N-acetylneuraminate

| Species | sex | $LD_{50}$ (mg/kg) | | | | |
|---|---|---|---|---|---|---|
| | | Administering route | | | | |
| | | Oral | Sub-cutaneous | Intra-peritoneal | Intravenous | Inhalation |
| Mice | male | >5,000 | >5,000 | >5,000 | >5,000 | - |
| | female | >5,000 | >5,000 | >5,000 | >5,000 | - |
| Rats | male | >5,000 | >5,000 | >5,000 | >5,000 | >4,000mg/$m^3$ |
| | female | >5,000 | >5,000 | >5,000 | >5,000 | >4,000mg/$m^3$ |
| Guinea pigs | | - | - | - | >5,000 | - |

Inhalation was proceeded by spraying the nebulized test compound for one hour.

[Example 3]

Simplified acute toxicity test:

A simplified acute toxicity test of N-acetylneuraminates in mice by intravenous injeciton was carried out as follows.

(1) Test compounds

Lithium-, potassium-, barium- and magnesium salts of N-acetylneuraminic acid were used (all were from MECT Corporation).

(2) Experimental animals

Male ddy mice
Body weights at the start of the test: 17.7-21.1 g
The number of animals at each level: 3 mice

(3) Room temperature: 23±1 °C Humidity: 55±7%

(4) Administering route

Vein

(5) Administering method and doses

The above-mentioned test compounds were dissolved in physiological saline solution to make 0.2 ml solution per 20 g of body weight in a mouse as an injection fluid and injected to the tail vein. The doses were 500, 1,000 and 2,000 mg/kg.

(6) Observation on general symptoms and death

Presence or absence of general symptoms and deaths were observed for 7 days immediately after administration.

(7) Lethality

The lethalities are shown in Table 4.

Table 4

The number of deaths with day course

| Drug | Dose mg/kg | 1 3 6 | 24 hrs | 2 3 4 5 6 | 7 days | Final Lethality |
|---|---|---|---|---|---|---|
| Lithium N-acetylneuraminate | 500 | 0 0 0 | 0 | 0 0 0 0 0 | 0 | 0/3 |
| | 1000 | 0 0 0 | 0 | 0 0 0 0 0 | 0 | 0/3 |
| | 2000 | 0 0 0 | 0 | 0 0 0 0 0 | 0 | 0/3 |
| Potassium N-acetylneuraminate | 500 | 0 0 0 | 0 | 0 0 0 0 0 | 0 | 0/3 |
| | 1000 | 3 0 0 | 0 | 0 0 0 0 0 | 0 | 3/3 |
| | 2000 | 3 0 0 | 0 | 0 0 0 0 0 | 0 | 3/3 |
| Barium N-acetylneuraminate | 500 | 3 0 0 | 0 | 0 0 0 0 0 | 0 | 3/3 |
| | 1000 | 3 0 0 | 0 | 0 0 0 0 0 | 0 | 3/3 |
| | 2000 | 3 0 0 | 0 | 0 0 0 0 0 | 0 | 3/3 |
| Magnesium N-acetylneuraminate | 500 | 0 0 0 | 0 | 0 0 0 0 0 | 0 | 0/3 |
| | 1000 | 3 0 0 | 0 | 0 0 0 0 0 | 0 | 3/3 |
| | 2000 | 3 0 0 | 0 | 0 0 0 0 0 | 0 | 3/3 |

At the doses of 1,000 and 2,000 mg/kg, all of the 3 mice died in the groups treated with magnesium-, barium- and potassium N-acetylneuraminates. At the dose of 500 mg/kg, 3 of the 3 mice in the group given barium N-acetylneuraminate died. There were no deaths in any other group.

(8) General symptoms

The death followed clonic convulsion and incontinence of urine and most of the animals died immediately or within one minute after injection. In a few survivors, inhibition of spontaneous movement was observed, which was normalized within one hour.

As mentioned above, N-acetylneuraminic acid has an inhibitory effect on nephritis and is an useful compound as a therapeutic for renal disease.

## Claims

**1.** A preparation for treating renal disease, which comprises, an effective amount of a compound shown by the formula:

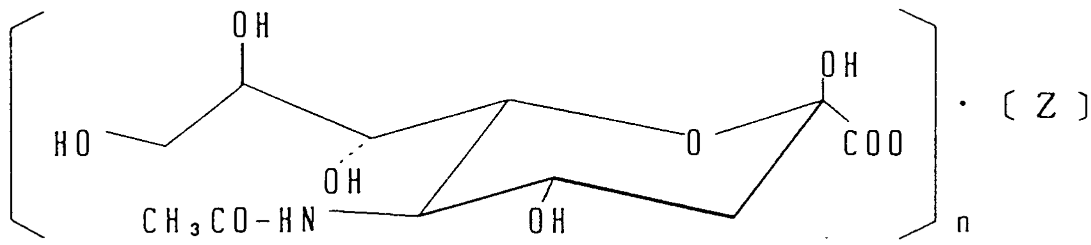

wherein, when n is 1, Z represents hydrogen, lithium, potassium, sodium, ammonium or oraganic ammonium, and, when n is 2, Z represents calcium, barium or magnesium; and, a pharmacologically acceptable carrier.

**2.** The preparation of claim 1, wherein:
n is 1 and Z represents sodium.

**3.** The preparation of claim 1, wherein:
said carrier is at least one agent selected from the group consisting of physiological saline solution, stabilizers or preservatives.

**4.** A method for treating renal disease, which comprises:
administerring the preparation containing an effective amount of a compound represented by the formula:

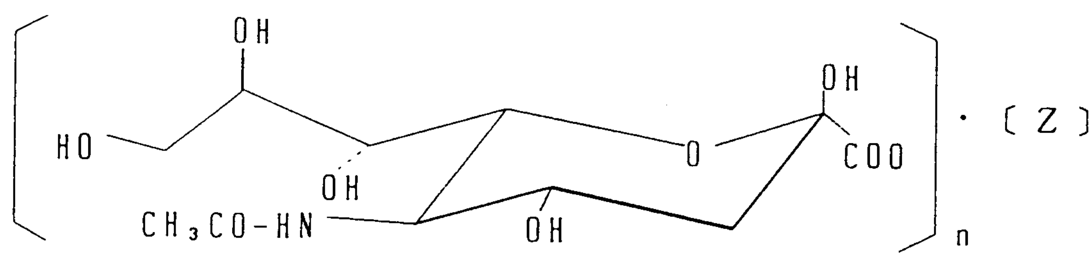

wherein, when n is 1, Z represents hydrogen, lithium, potassium, sodium, ammonium or oraganic ammonium, and, at when n is 2, Z represents calcium, barium or magnesium;
and, a pharmacologically acceptable carrier.

**5.** The method of claim 4, wherein:
said preparation is administered by injection means.

**6.** The method of claim 4, wherein:
said injection means is intraperitoneal, subcutaneous or intravenous.

**7.** The method of claim 4, wherein:
the preparation also contains at least one agent as a carrier selected from the group consisting of physiological saline solution, stabilizers and preservatives.

FIG. 1

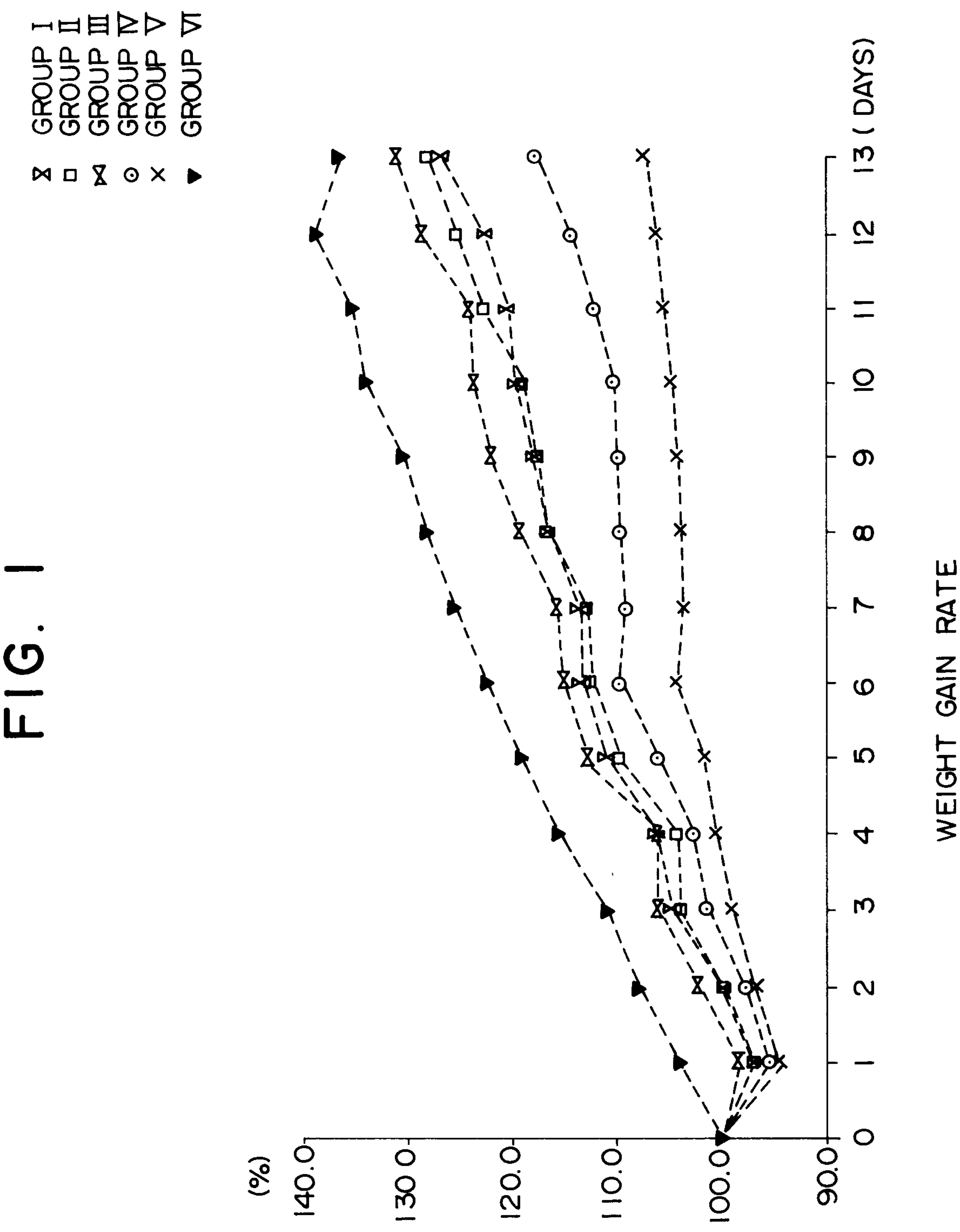
GROUP I
GROUP II
GROUP III
GROUP IV
GROUP V
GROUP VI
(%)
140.0
130.0
120.0
110.0
100.0
90.0
0
1
2
3
4
5
6
7
8
9
10
11
12
13 (DAYS)
WEIGHT GAIN RATE

FIG. 2

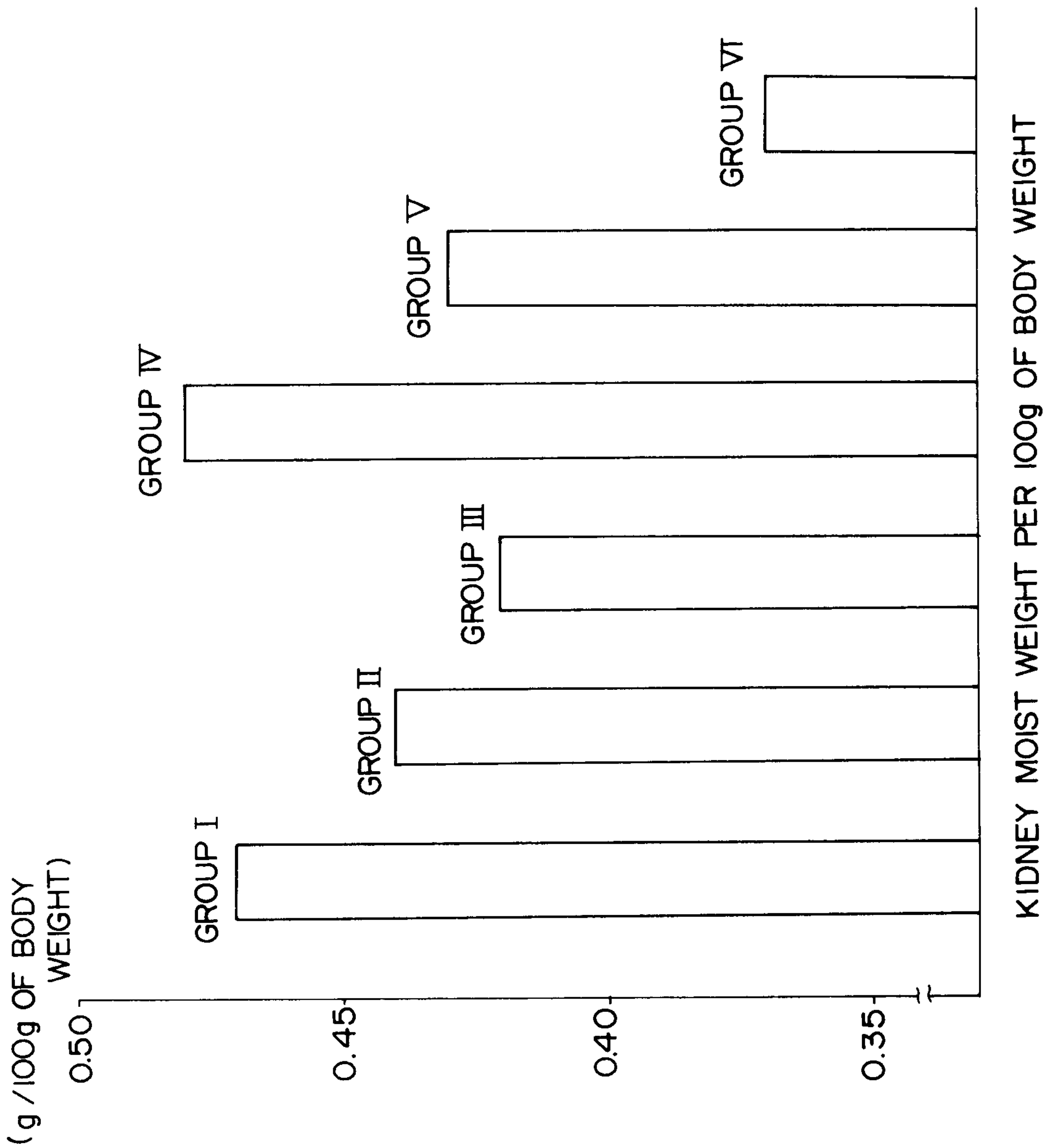
(g /100g OF BODY WEIGHT)
0.50
0.45
0.40
0.35
GROUP I
GROUP II
GROUP III
GROUP IV
GROUP V
GROUP VI
KIDNEY MOIST WEIGHT PER 100g OF BODY WEIGHT

FIG. 3

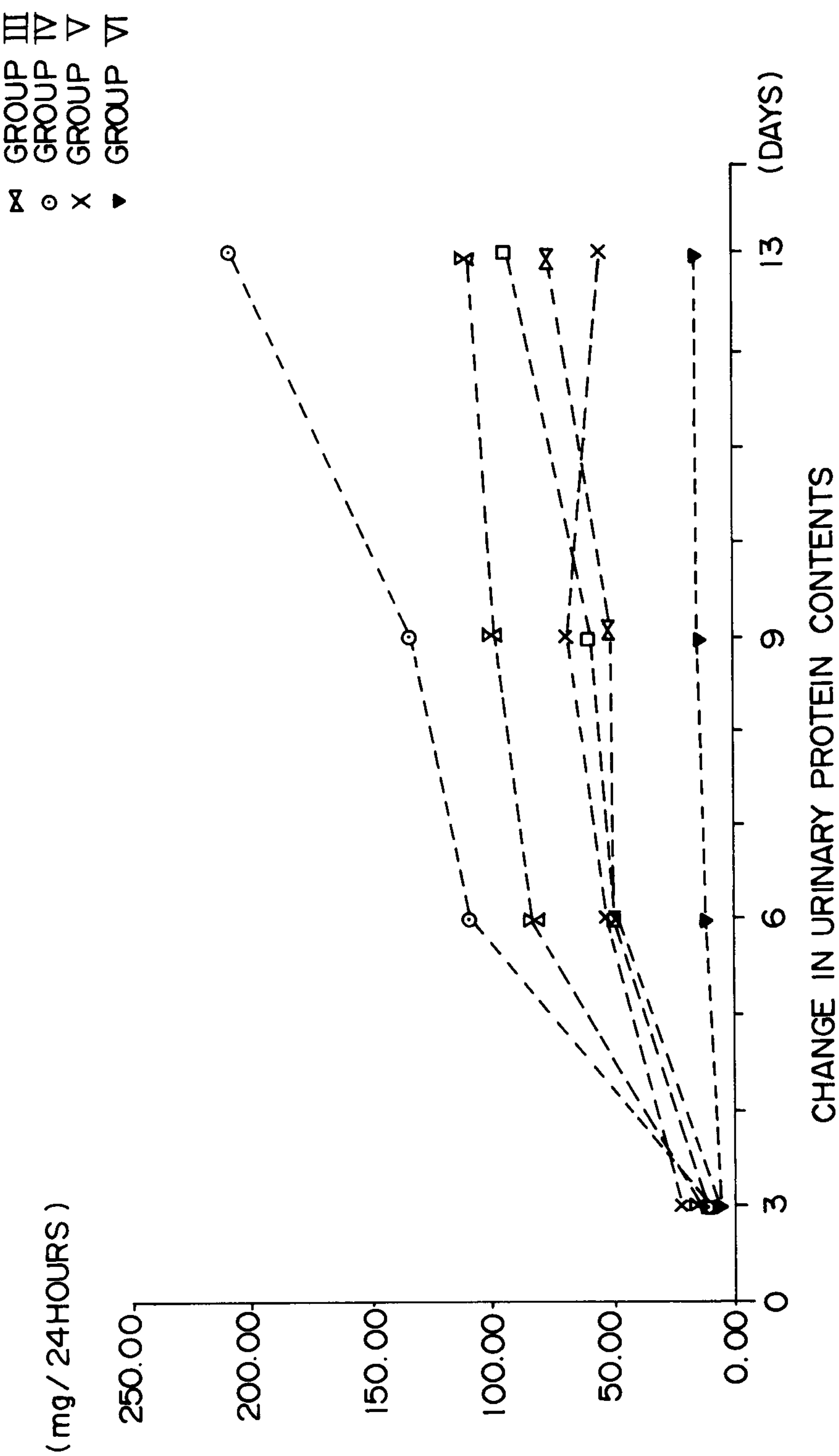
(mg/24HOURS)
250.00
200.00
150.00
100.00
50.00
0.00
0
3
6
9
13
(DAYS)
CHANGE IN URINARY PROTEIN CONTENTS
GROUP I
GROUP II
GROUP III
GROUP IV
GROUP V
GROUP VI